# EUROPÄISCHE PATENTANMELDUNG

(11) **EP 4 703 347 A1**
(43) Veröffentlichungstag der Anmeldung: **04.03.2026**
(21) Anmeldenummer: 24196930.2
(22) Anmeldetag: 28.08.2024
(51) Int. Cl.: C07C 69/54, C07C 67/343, C07C 67/38, C07C 69/24, C07C 1/22, C07C 11/04, C25B 3/26, C25B 1/23, C08F 20/14

(54) **POLYMETHYLMETHACRYLAT AUS CO2 DER ATMOSPHÄRE**

(71) Anmelder: VOLKSWAGEN AG, 38440 Wolfsburg (DE)
(72) Erfinder: Aniol, Armin, 38124 Braunschweig (DE); Fischer, Fabian, 30449 Hannover (DE)

(57) **Zusammenfassung**

Vorliegend wird ein Verfahren zur Herstellung von Methylmethacrylat umfassend die Schritte Vorlegen von Ethylen, Vorlegen von Kohlenstoffmonooxid, Vorlegen von Methanol, Umsetzen des Ethylens, des Kohlenstoffmonooxids und des Methanols zu Methylmethacrylat, wobei das Methylmethacrylat Kohlendioxid aus der Atmosphäre umfasst. Ferner werden ein Verfahren zur Herstellung von Polymethylmethacrylat, Methylmethacrylat, Polymethylmethacrylat, sowie die Verwendung von Methylmethacrylat bzw. Polymethylmethacrylat beschrieben.

## Beschreibung

Die Erfindung betrifft ein Verfahren zur Herstellung von Methylmethacrylat, ein Verfahren zur Herstellung von Polymethylmethacrylat, Methylmethacrylat, Polymethylmethacrylat sowie die Verwendung von Methylmethacrylat und Polymethylmethacrylat.

Zur Verbesserung der Gesamt-CO₂-Bilanz von Fahrzeugen ist der Einsatz von nachhaltigen Werkstoffen ein effektiver Stellhebel. Nachhaltige Polymerlösungen besitzen in diesem Zuge in der Automobilindustrie eine immer stärkere Bedeutung. Die drei relevantesten Rohstoffquellen für nachhaltige Polymere umfassen hierbei biobasierte Ansätze auf Basis nachwachsender Rohstoffe, Rezyklatkunststoffe sowie CO₂-basierte Polymeransätze.

In der Literatur sind bereits eine Vielzahl von biobasierten Polymeren bekannt (PLA, PHB, etc.), um den CO₂-Fußabdruck über den gesamten Produktlebenszyklus im Vergleich zur petrochemischen Alternative gering zu halten. Weiterhin werden vermehrt Polymere aus Rezyklatprozessen eingesetzt, um den CO₂-Fußabdruck durch einen geschlossenen Werkstoffkreislauf gering zu halten. Diese beiden Polymerklassen sind häufig jedoch nicht für den Einsatz in Anwendungen mit anspruchsvollen Anforderungen geeignet, da sowohl bei den biobasierten Ansätzen als auch bei den Rezyklatkunststoffen einerseits durch natürliche Syntheseprozesse und andererseits durch Degradationseffekte im Recyclingprozess kein definiertes Molekulargewicht erhalten werden kann und damit eine Varianz der physikalischen, mechanischen und chemischen Eigenschaften auftritt.

Polymere auf Basis von CO₂ besitzen eine definierte Molekulargewichtsverteilung und somit ein, auf die jeweilige Anwendung spezifisches Eigenschaftsprofil und unterscheiden sich chemisch in der Regel nicht von petrochemischen Kunststoffansätzen. Der größte Vorteil gegenüber petrochemischen Polymerlösungen ist eine bessere CO₂-Bilanz.

Polymethylmethacrylat (PMMA) ist ein amorpher Thermoplast, der durch Kettenpolymerisation von Methacrylsäuremethylester hergestellt wird.

PMMA kann durch radikalische Substanz-, Emulsions- oder Suspensionspolymerisation von Monomeren fossilen Ursprungs hergestellt werden. Die Ausbildung polymerer Strukturen aus fossilen Ressourcen besitzt u.a. die Nachteile begrenzte Ressourcen in polymere Strukturen einzubauen sowie eine Abhängigkeit von globalen Erdölquellen aufzuweisen. Des Weiteren besitzt die petrochemische Kunststoffvariante einen höheren CO₂-Fußabdruck.

Calvinho et al. beschreiben verschiedene Nickel-Phosphide, mit denen CO₂ in wässriger Lösung zu C₃-C₄-Verbindungen, wie 2,3-Furandiol (C₄) und das Nebenprodukt Methylglyoxal (C₃), umgesetzt werden kann (Calvinho et al., Energy Environ. Sci., 2018, 11, 2550-2559).

US 2020/0347502 A1 beschreibt ebenfalls Nickelphosphide zur elektrochemischen Reduktion von CO₂ zu Kohlenwasserstoffen unter Verwendung von Nickelphosphid-Nanopartikeln.

Lopez et al. beschreiben die elektrochemische Corey-Winter-Eliminierungsreaktion mit Trimethylphosphit oder Triethylphosphit oder dem bekannten Corey-Winter-Reagenz (Lopez et al., Beilstein H. Org. Chem. 2018, 14, 547-552).

Die Herstellung von n-Butan mithilfe einer übergangsmetallkatalysierten Reaktion wird ebenfalls in der Literatur offenbart (Fujimoto et. al. Direct synthesis of propane/butane from synthesis gas. In: Studies in surface science and catalysis. Elsevier, 2007. S. 349-354).

Die Herstellung von 1,3-Butadien aus petrochemischen Quellen sowie die Umsetzung von n-Butan aus CO₂ über alternative katalytische Synthesewege wird u.a. in Hua, Yani, et al. Electrochemical CO2 conversion towards syngas: Recent catalysts and improving strategies for ratio-tunable syngas. Journal of Power Sources, 2022, 535. Jg., S. 231453 beschrieben.

Die Herstellung von PMMA aus fossilen Grundstoffen erfolgt in der Regel nach mehreren Schritten. Dabei können die einzelnen Monomerverbindungen im industriellen Maßstab aus fossilen Rohstoffen nach folgendem Schema hergestellt werden:
Die Synthese des Ethylens erfolgt mittels katalytischem oder thermischem Cracken von Kohlenwasserstoffverbindungen aus Erdöl sowie teilweise als Koppelprodukt/Nebenprodukt der chemischen Industrie.

Die konventionelle Synthese von Kohlenstoffmonooxid erfolgt mithilfe der Kohlevergasung. Hierzu entsteht aus Kohle und Wasser in einer Verbrennungsreaktion Kohlenstoffmonooxid sowie Wasserstoff (Synthesegas). Die Reaktion verläuft hierbei endotherm in einer Gleichgewichtsreaktion (Boudouard-Gleichgewicht) ab.

Die Synthese des Methanols verläuft über die Synthese des Synthesegases. Hierzu entsteht aus Kohle und Wasser in einer Verbrennungsreaktion Kohlenstoffmonooxid sowie Wasserstoff (Synthesegas). Die Reaktion verläuft hierbei endotherm in einer Gleichgewichtsreaktion (Boudouard-Gleichgewicht) ab. Die anschließende technische Herstellung von Methanol erfolgt fast ausschließlich in katalytischen Verfahren aus Synthesegas (Verhältnis Kohlenstoffmonooxid und Wasserstoff 1:2). Hierbei können, je nach Druckbereich der Reaktion, unterschiedliche Verfahren angewendet werden: Das Hochdruckverfahren verläuft bei Drücken von 250 bis 350 bar und Temperaturen von 360 bis 380 °C. Das Mitteldruckverfahren verläuft bei 100 bis 250 bar und Temperaturen von 220 bis 300 °C. Das Niederdruckverfahren verläuft bei 50 bis 100 bar und 200 bis 300 °C.

Die Synthese des Methylmethacrylates kann nach dem industriellen Alpha-Verfahren erfolgen. Hierbei geht das Verfahren von Ethylen als Rohstoff aus, das homogen katalysiert mit Kohlenmonoxid und Methanol in einem Schritt carboxymethyliert wird. Hierbei entsteht Propionsäuremethylester als Zwischenprodukt. Die Propionsäuremethylesterverbindung wird im nächsten Schritt in der Gasphase an einem speziellen Kontakt mit Formaldehyd aldolisiert und dehydratisiert, wobei direkt MMA entsteht.

PMMA wird großtechnisch meist radikalisch durch Substanz-, Emulsions-, oder Suspensionspolymerisation hergestellt. Auf solche Weise produziertes PMMA ist ataktisch und völlig amorph. Die Polymerisation wird mit sogenannten Radikalbildnern wie beispielsweise Dibenzoylperoxid (DBPO) oder Azo-bis-(isobutylonitril) (AiBN) initiiert. Folgend verläuft das Kettenwachstum radikalisch bis Rekombinationsreaktionen eintreten. Eine anionische Polymerisation (einschließlich Methoden der lebenden Polymerisation) von PMMA ist ebenfalls möglich.

Aufgabe der vorliegenden Erfindung ist es, ein Verfahren zur Herstellung von Methylmethacrylat, ein Verfahren zur Herstellung von Polymethylmethacrylat, Methylmethacrylat, Polymethylmethacrylat und die Verwendung von Methylmethacrylat und Polymethylmethacrylat bereitzustellen, das bzw. die die oben genannten Nachteile wenigstens teilweise überwindet.

Diese Aufgabe wird durch das erfindungsgemäße Verfahren nach Anspruch 1, das erfindungsgemäße Verfahren nach Anspruch 2, Methylmethacrylat und Polymethylmethacrylat nach Anspruch 11 und die Verwendung nach Anspruch 12 gelöst.

Weitere vorteilhafte Ausgestaltungen der Erfindung ergeben sich aus den Unteransprüchen und der folgenden Beschreibung bevorzugter Ausführungsbeispiele der vorliegenden Erfindung.

Ein erfindungsgemäßes Verfahren zur Herstellung von Methylmethacrylat umfasst die Schritte:
- Vorlegen von Ethylen,
- Vorlegen von Kohlenstoffmonooxid,
- Vorlegen von Methanol, und
- Umsetzen des Ethylens, des Kohlenstoffmonooxids und des Methanols zu Methylmethacrylat,
wobei das Methylmethacrylat Kohlendioxid aus der Atmosphäre umfasst.

In einem erfindungsgemäßen Verfahren umfasst das Methylmethacrylat CO₂ aus der Atmosphäre. Insbesondere durch die Verwendung von CO₂ aus der Atmosphäre bzw. aus nachhaltigen Quellen, wie der thermischen Verwertung biogener Abfallströme, kann die CO₂-Bilanz gesenkt werden.

Der Einsatz von CO₂ aus der Atmosphäre führt im Vergleich zum Einsatz von CO₂ aus fossilen Rohstoffen zu einem geringeren CO₂-Fußabdruck des Verfahrensprodukts, d.h. z. B. dem Polymethylmethacrylat.

Die Ausbildung polymerer Strukturen aus fossilen Ressourcen besitzt den Nachteil, begrenzte Ressourcen in polymere Strukturen einzubauen sowie eine Abhängigkeit von globalen Erdölquellen aufzuweisen.

In einer Ausführungsform ist das Methanol aus CO₂ aus der Atmosphäre gebildet.

Zunächst wird im erfindungsgemäßen Verfahren Ethylen vorgelegt. Bei dem Ethylen kann es sich um Ethylen aus fossilen Quellen, oder um Ethylen aus nachhaltigen Quellen handeln. Vorzugsweise wird Ethylen verwendet, das aus CO₂ aus der Atmosphäre gewonnen wird. So kann der CO₂-Fußabdruck des Endprodukts weiter gesenkt werden.

In einer Ausführungsform umfasst der Schritt des Vorlegens von Ethylen den Schritt des elektrochemischen Reduzierens von Kohlendioxid zu Ethylen. Mit anderen Worten ist das eingesetzte Ethylen durch die elektrochemische Reduktion von CO₂ erhalten worden. Beim elektrochemischen Reduzieren wird das CO₂ unter Bildung von Monoethylenglycol reduziert. Bei der elektrochemischen Reduktion wird das CO₂ somit durch Zufuhr von elektrischer Energie reduziert. Vorzugsweise handelt es sich bei der elektrochemischen Reduktion des CO₂ um eine katalytische elektrochemische Reduktion. Dabei wird unter Verwendung eines Katalysators die Energie gesenkt, die bei der elektrochemischen Reduktion aufgewendet werden muss. Bei dem Katalysator kann es sich um einen heterogenen, oder homogenen Katalysator handeln. Die elektrochemische Reduktion des CO₂ findet vorzugsweise durch die Anlagerung des CO₂ über die Sauerstoffatome unter einer gleichzeitigen Hydridanbindung an eine katalytisch aktive Oberfläche statt. Durch eine Protonierung dieser Spezies kann Formaldehyd als Zwischenprodukt generiert werden. Das so entstandene Formaldehyd kann im Anschluss mit einer weiteren Formaldehydspezies über eine C-C-Kupplungsreaktion an der katalytisch aktiven Elektrodenoberfläche zu einem Glycoaldehyd reagieren. Dieses kann unter Reduktion mittels Hydridanbindung zur intermediären Alkoholatspezies reagieren, welche im Anschluss protoniert in wässriger Lösung Monoethylenglykol (MEG) ausbilden kann.

Bei dem Katalysator kann es sich um einen Übergangsmetallkatalysator handeln. Bei den Übergangsmetallen kann es sich um Nickel, Eisen, Kupfer oder Molybdän handeln. Bei den Übergangsmetallverbindungen kann es sich um geeignete Verbindungen, wie Phosphide, Sulfide oder Oxide handeln. Beispielhafte Übergangsmetallverbindungen, die als Katalysatoren fungieren können, sind Nickelphosphid (Ni₂P) oder Eisenphosphid (Fe₂P).

Bevorzugt umfasst der Katalysator der katalytischen induzierten elektrochemischen Reduktion Ni₂P. Weiter bevorzugt umfasst der Katalysator der katalytischen induzierten elektrochemischen Reduktion eine Kombination aus Katalysator und Co-Katalysator, z. B. Ni₂P/Fe₂P.

Bevorzugt ist der Einsatz einer Mischung aus Nickel-Phosphid (Ni-P) und Eisen-Phosphid (Fe-P) als Katalysator. Besonders bevorzugt ist der Einsatz einer Mischung aus Ni₂P mit Fe₂P als Katalysator. Das Massenverhältnis von Ni₂P : Fe₂P liegt zwischen 99 : 1 Gew.-% und 1 : 99 Gew.-%, bevorzugt zwischen 95 : 5 Gew.-% und 5 : 95 Gew.-%, besonders bevorzugt zwischen 90 : 10 Gew.-% und 10 : 90 Gew.-%, ganz besonders bevorzugt zwischen 25 : 75 Gew.-% und 75 : 25 Gew.-%.

Für die elektrochemische Reduktion ist es denkbar, dass eine Reihe von Elektroden eingesetzt werden können. Beispielhafte Elektroden sind Redox-Elektroden, lonenselektive Elektroden, Gasdiffusionselektroden, etc.. Besonders vorteilhaft wird in einem erfindungsgemäßen Verfahren eine Gasdiffusionselektrode eingesetzt. Ferner kann die Umsetzung des Monoethylenglycols zu Ethylen stattfinden. Bevorzugt findet die Umsetzung des Monoethylenglycols zu Ethylen unter Corey-Winter-Eliminierung statt.

Dabei kann es sich um eine elektrochemische Corey-Winter-Eliminierung, oder um eine nasschemische Corey-Winter-Eliminierung handeln. Bevorzugt handelt es sich in einem erfindungsgemäßen Verfahren um eine nasschemische Corey-Winter-Eliminierung. Bei der nasschemischen Corey-Winter-Eliminierung wird vorzugsweise das sogenannte Corey-Hopkins-Reagenz (1,3-Dimethyl-2-phenyl-1,3,2-diazaphospholidin) für die Umsetzung der Edukte eingesetzt.

Bei einer elektrochemischen Corey-Winter-Eliminierung wird anstelle des Corey-Winter Reagenz Trimethylphosphit bei 111 °C oder eine Potentio(Reticulated Vitreous Carbons = Glaskohlenstoff)- Kathode, für 1,5 Stunden eingesetzt. Bevorzugt handelt es sich in einem erfindungsgemäßen Verfahren um eine nasschemische Corey-Winter-Eliminierung. Bei der Corey-Winter Eliminierung, oder auch Corey-Winter-Fragmentierung genannt, handelt es sich um eine Olefinsynthese durch Eliminierung vicinaler Diole. Beispielhaft wird bei einer nasschemischen Corey-Winter-Eliminierung ein Diol mit Thiophosgen in Anwesenheit von 4-Dimethylaminopyridin (DMAP) zu einem cyclischen Thionocarbonat umgesetzt. Das Thionocarbonat kann anschließend in Gegenwart einer trivalenten Phosphorverbindung zu einem Olefin umgesetzt werden. Bei der trivalenten Phosphorverbindung kann es sich um Trimethylphosphit handeln.

Ein erfindungsgemäßes Verfahren umfasst weiter den Schritt des Vorlegens von Kohlenstoffmonooxid. Bei dem Kohlenstoffmonooxid kann es sich um Kohlenstoffmonooxid aus fossilen Quellen, oder um Kohlenstoffmonooxid aus nachhaltigen Quellen handeln. Vorzugsweise wird Kohlenstoffmonooxid verwendet, das aus CO₂ aus der Atmosphäre gewonnen wird. So kann der CO₂-Fußabdruck des Endprodukts weiter gesenkt werden.

In einer Ausführungsform umfasst das Vorlegen von Kohlenstoffmonooxid den Schritt des elektrochemischen Reduzierens von Kohlendioxid zu Kohlenstoffmonooxid. Mit anderen Worten ist das eingesetzte Kohlenstoffmonooxid durch die elektrochemische Reduktion von CO₂ erhalten worden.

Die Umsetzung des Kohlenstoffdioxids zu Kohlenstoffmonooxid kann mithilfe übergangsmetallkatalysierter Reaktionen erfolgen. Die CO₂-Reduktion zum Kohlenstoffmonooxid kann hierbei anhand dem Stand der Technik (HUA, Yani, et al. Electrochemical CO2 conversion towards syngas: Recent catalysts and improving strategies for ratiotunable syngas. Journal of Power Sources, 2022, 535. Jg., S. 231453) erfolgen. Die bevorzugte Variante besteht hierbei aus einer Gasdiffusionselektrode mit einem Elektrokatalysator, bestehend aus mindestens einem der folgenden Elemente: Zn, Co, Cr, Ni, Cu und Fe.

Ein erfindungsgemäßes Verfahren umfasst weiter den Schritt des Vorlegens von Methanol. Das Methanol umfasst vorzugsweise CO₂ aus der Atmosphäre. Alternativ kann das Methanol aus CO₂ aus der Atmosphäre gebildet sein.

Die Umsetzung von CO₂ zu Methanol kann über verschiedene Zwischenstufen erfolgen. Das CO₂ kann mittels Wasserstoff zu einem intermediären Kohlenstoffmonooxid reduziert werden und anschließend durch weitere Zugabe von Wasserstoff zu Methanol reduziert werden. Die Reduktion kann alternativ mittels Katalyse erfolgen. Die Reduktion kann z. B. übergangsmetallkatalysiert, oder direkt elektrochemisch mit geeigneten Elektrokatalysatoren erfolgen ohne ein intermediäres Kohlenstoffmonooxid auszubilden. Bei dem Katalysator kann es sich um einen Übergangsmetallkatalysator handeln. Der Katalysator kann dabei ausgewählt sein aus der Gruppe bestehend aus Platin-, Nickel-, Eisen-, Silber-, Kupfer-Katalysatoren und Kombinationen davon. Beispielsweise kann der Katalysator ausgewählt sein aus der Gruppe bestehend aus Platin-, Nickel-, Eisen-, Silber-, und Kupferbasierten Phosphorverbindungen.

In einem weiteren Schritt des erfindungsgemäßen Verfahrens erfolgt das Umsetzen des Ethylens, des Kohlenstoffmonooxids und des Methanols zu Methylmethacrylat.

Das Umsetzen des Ethylens, des Kohlenstoffmonooxids und des Methanols zu Methylmethacrylat kann in einem bestimmten Verhältnis erfolgen. Das Verhältnis von Ethylen: Kohlenstoffmonooxid:Methanol erfolgt vorzugsweise im äquimolaren Verhältnis von 1:1:1.

In einer Ausführungsform erfolgt das Umsetzen des Ethylens, des Kohlenstoffmonooxids und des Methanols zu Methylmethacrylat unter heterogener Katalyse. Dabei kann der Katalysator ausgewählt sein aus der Gruppe bestehend aus Palladium- sowie Platinverbindungen mit Phosphin-Liganden.

Die Synthese des Methylmethacrylates kann nach dem industriellen Alpha-Verfahren erfolgen. Hierbei geht das Verfahren von Ethylen als Rohstoff aus, das homogen katalysiert mit Kohlenstoffmonooxid und Methanol in einem Schritt carboxymethyliert wird. Hierbei entsteht Propionsäuremethylester als Zwischenprodukt. Die Propionsäuremethylesterverbindung wird im nächsten Schritt in der Gasphase an einem speziellen Kontakt mit Formaldehyd aldolisiert und dehydratisiert, wobei direkt MMA entsteht.

In einer Ausführungsform erfolgt das Umsetzen des Ethylens, des Kohlenstoffmonooxids und des Methanols unter Ausbildung von Propionsäuremethylester als Zwischenprodukt.

In einer weiteren Ausführungsform wird der Propionsäuremethylester mit Formaldehyd zu Methylmethacrylat umgesetzt.

Weiterhin ist Gegenstand der vorliegenden Erfindung ein Verfahren zur Herstellung von Polymethylmethacrylat umfassend die Schritte:
- Herstellen von Methylmethacrylat nach einem erfindungsgemäßen Verfahren und
- Polymerisieren des Methylmethacrylats zu Polymethylmethacrylat.

Die Ausbildung des Polymethylmethacrylats (PMMA) in einem erfindungsgemäßen Verfahren erfolgt durch das Polymerisieren des Methylmethacrylats (MMA).

Das PMMA kann großtechnisch radikalisch durch Substanz-, Emulsions-, oder Suspensionspolymerisation hergestellt werden. Auf solche Weise produziertes PMMA ist in der Regel ataktisch und völlig amorph. Die Polymerisation kann mit sogenannten Radikalbildnern wie beispielsweise Dibenzoylperoxid (DBPO) oder Azo-bis-(isobutylonitril) (AiBN) initiiert werden. Folgend verläuft das Kettenwachstum vorzugsweise radikalisch bis Rekombinationsreaktionen eintreten. Eine anionische Polymerisation (einschließlich Methoden der lebenden Polymerisation) von PMMA ist ebenfalls möglich.

In einem erfindungsgemäßen Verfahren werden Ethylen, Kohlenstoffmonooxid und Methanol vorzugsweise aus CO₂ aus der Atmosphäre gebildet.

Ein erfindungsgemäßes Verfahren kann ferner den Schritt der Isolation von CO₂ aus der Atmosphäre umfassen.

Ferner ist Gegenstand der vorliegenden Erfindung Methylmethacrylat bzw. Polymethylmethacrylat hergestellt nach einem erfindungsgemäßen Verfahren. Durch den Einsatz von CO₂ aus der Atmosphäre bei der Herstellung von Methylmethacrylat bzw. Polymethylmethacrylat, kann ein Polymer mit geringerem CO₂-Fußabdruck als ein Polymer mit CO₂ aus fossilen Quellen hergestellt werden.

Weiterhin ist Gegenstand der vorliegenden Erfindung die Verwendung von Methylmethacrylat bzw. Polymethylmethacrylat in der Automobilindustrie.

In einer Ausführungsform wird das Methylmethacrylat bzw. Polymethylmethacrylat in Blinker- und Rückleuchtengläser, Reflektoren, Lichtleiter und Tür-/Säulenverkleidungen im Exterieur- und Interieurbereich (z. B. Verkleidung von A-/B-/C-Säulen) verwendet.

In einer weiteren Ausführungsform kann das Methylmethacrylat bzw. Polymethylmethacrylat in Polymerbeton, Industriefußböden, Verglasungen (z. B. Doppelstegplatten), Detailabdichtungen im Flachdach, Industrietorverglasungen (Plustherm-Systemverglasung), Sanitär- und Einrichtungsbauteile, Türfüllungen, Lampenschirme, Verwendung als Resist (Fotolack) bzw. Bestandteil davon in der Foto- und Elektronenstrahllithographie zur Herstellung von Schaltkreisen und Leiterplatten, Flutlicht-Schilder, Leuchtenabdeckungen, Leuchtwerbung, Schauglas, Linsen, Fresnel-Linsen, Lichtwellenleiter, Scheiben, Hauben, Scheinwerferabdeckungen, Methylmethacrylatklebstoff für Verbindungen von Metallen und Kunststoffen, Schlagzeuge und Tastenbeläge von Klavieren verwendet werden.

Ein erfindungsgemäßes Verfahren kann eine Drop-In-Lösung für MMA-herstellende, PMMAherstellende, MMA-verarbeitende, PMMA-verarbeitende Industriezweige sowie Endanwender darstellen.

Ausführungsbeispiele der Erfindung werden nun beispielhaft und unter Bezugnahme auf die beigefügte Zeichnung beschrieben, in der:
Fig. 1 schematisch ein Ausführungsbeispiel eines erfindungsgemäßen Verfahrens,
Fig. 2a schematisch die Bildung von MMA nach dem Stand der Technik,
Fig. 2b schematisch die Umsetzung von MMA zu PMMA,
Fig. 3a schematisch ein Ausführungsbeispiel eines erfindungsgemäßen Verfahrens,
Fig. 3b schematisch die Umsetzung von MMA zu PMMA,
Fig. 4 ein strömungsgerechtes Profil einer Gasdiffusionselektrode und
Fig. 5 ein strömungsgerechtes Profil einer Gasdiffusionselektrode, zeigen.

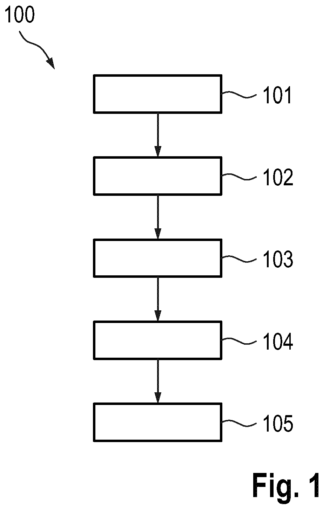

Fig. 1 zeigt schematisch eine Ausführungsform eines Verfahrens 100 zur Herstellung von Polymethylmethacrylat. Die Schritte 101, 102 und 103 bilden eine Ausführungsform eines Verfahrens zur Herstellung von Methylmethacrylat. Die Schritte 101, 102, 103 und 104 bilden eine Ausführungsform eines Verfahrens zur Herstellung von Polymethylmethacrylat 100. Zunächst wird Ethylen vorgelegt 101. Weiterhin wird Kohlenstoffmonooxid vorgelegt 102. In einem weiteren Schritt wird Methanol vorgelegt 103. Weiter wird das Ethylen, das Kohlenstoffmonooxid und das Methanol zu Methylmethacrylat umgesetzt 104 und anschließend das Methylmethacrylat zu Polymethylmethacrylat polymerisiert 105. Die einzelnen Komponenten können gleichzeitig, oder nacheinander in beliebiger Reihenfolge vorgelegt werden. Das Methanol umfasst vorzugsweise Kohlendioxid aus der Atmosphäre.

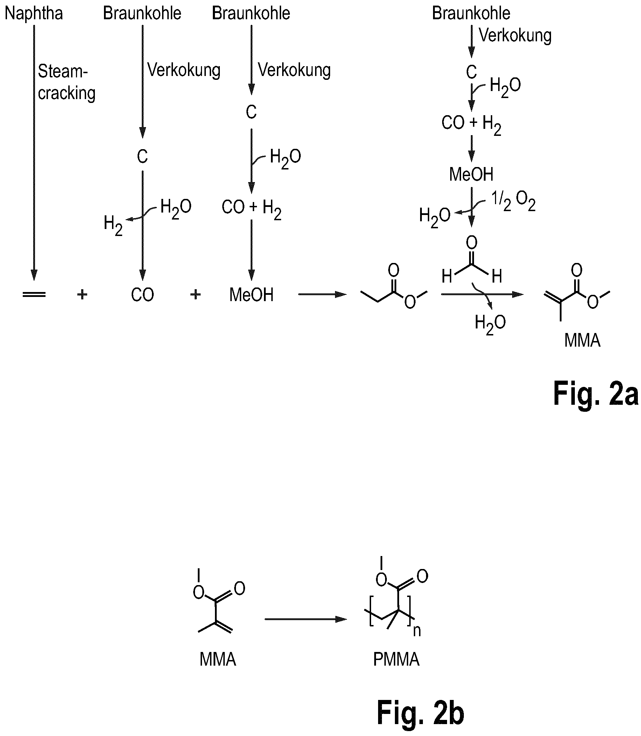

Fig. 2a zeigt schematisch die Bildung von MMA nach dem Stand der Technik. Ethylen wird aus Naphtha über Steam-cracking gebildet. Kohlenstoffmonooxid wird aus Braunkohle hergestellt. Dabei wird Braunkohle zunächst verkokt und so Kohlenstoff gebildet. Aus Wasser wird Wasserstoff gebildet, der mit dem Kohlenstoff zu Kohlenstoffmonooxid umgesetzt wird. Das Methanol wird durch verkoken von Braunkohle und anschließendem Umsetzen des gebildeten Kohlenstoffs mit Wasser zu Kohlenstoffmonooxid und Wasserstoff gebildet. Das so erhaltene Ethylen, das Kohlenstoffmonooxid und das Methanol reagieren zu Propionsäuremethylester. Der Propionsäuremethylester wird mit Formaldehyd zu MMA umgesetzt. Das Formaldehyd kann durch ein mehrstufiges Verfahren hergestellt werden. Dabei wird Braunkohle zu Kohlenstoff verkokt. Der Kohlenstoff wird mit Wasser zu Kohlenstoffmonooxid und Wasserstoff umgesetzt, aus dem Methanol entsteht. Das Methanol reagiert mit Sauerstoff unter Abspaltung von Wasser zu Formaldehyd.

Fig. 2b zeigt schematisch die Umsetzung von MMA zu PMMA. Dabei wird MMA in einer radikalischen Reaktion mit Dibenzoylperoxid (DBPO) oder Azo-bis-(isobutylonitril) (AiBN) zu PMMA umgesetzt.

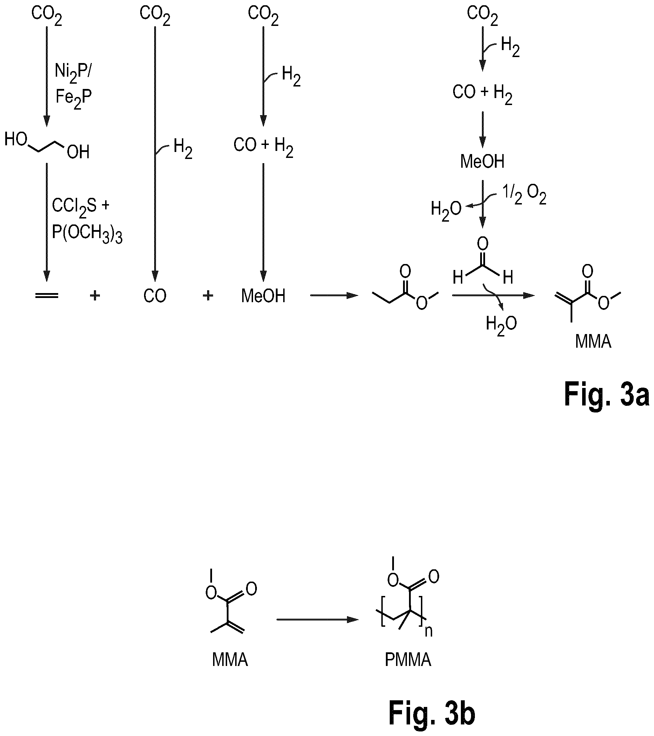

Fig. 3a zeigt schematisch die Bildung von MMA nach einer Ausführungsform eines erfindungsgemäßen Verfahrens. Das CO₂ wird unter Bildung von Monoethylenglycol elektrochemisch reduziert. Bei der katalytischen elektrochemischen Reduktion wird das CO₂ durch Zufuhr von elektrischer Energie reduziert. Bei dem Katalysator kann es sich in dieser Ausführungsform um einen Ni₂P/Fe₂P-Katalysator handeln. Die elektrochemische Reduktion des CO₂ findet vorzugsweise durch die Anlagerung des CO₂ über die Sauerstoffatome unter einer gleichzeitigen Hydridanbindung an die katalytisch aktive Oberfläche statt. Durch eine Protonierung dieser Spezies kann Formaldehyd als Zwischenprodukt generiert werden. Das so entstandene Formaldehyd kann im Anschluss mit einer weiteren Formaldehydspezies über eine C-C-Kupplungsreaktion an der katalytisch aktiven Elektrodenoberfläche zu einem Glycoaldehyd reagieren. Dieses kann unter Reduktion mittels Hydridanbindung zur intermediären Alkoholatspezies reagieren, welche im Anschluss protoniert in wässriger Lösung Monoethylenglykol (MEG) ausbilden kann. Für die elektrochemische Reduktion ist es denkbar, dass eine Reihe von Elektroden eingesetzt werden können. Beispielhafte Elektroden sind Redox-Elektroden, lonenselektive Elektroden, Gasdiffusionselektroden, etc.. Besonders vorteilhaft wird in einem erfindungsgemäßen Verfahren eine Gasdiffusionselektrode eingesetzt. Ferner umfasst ein erfindungsgemäßes Verfahren die Umsetzung des Monoethylenglycols zu Ethylen. Die Umsetzung des Monoethylenglycols zu Ethylen erfolgt in der vorliegenden Ausführungsform unter einer Corey-Winter-Eliminierung. Bevorzugt handelt es sich in einem erfindungsgemäßen Verfahren um eine nasschemische Corey-Winter-Eliminierung. Bei der Corey-Winter Eliminierung, oder auch Corey-Winter-Fragmentierung genannt, handelt es sich um eine Olefinsynthese durch Eliminierung vicinaler Diole. Beispielhaft wird bei einer nasschemischen Corey-Winter-Eliminierung ein Diol mit Thiophosgen in Anwesenheit von 4-Dimethylaminopyridin (DMAP) zu einem cyclischen Thionocarbonat umgesetzt. Das Thionocarbonat kann anschließend in Gegenwart einer trivalenten Phosphorverbindung zu einem Olefin umgesetzt werden. Bei der trivalenten Phosphorverbindung kann es sich um Trimethylphosphit handeln.

Die Umsetzung des Kohlenstoffdioxids zu Kohlenstoffmonooxid erfolgt in der vorliegenden Ausführungsform mithilfe übergangsmetallkatalysierter Reaktionen. Die CO₂-Reduktion mit Wasserstoff zum Kohlenstoffmonooxid erfolgt hierbei mit Gasdiffusionselektrode mit einem Elektrokatalysator, bestehend aus mindestens einem der folgenden Elemente: Zn, Co, Cr, Ni, Cu und Fe.

Die Bildung von Methanol erfolgt in der vorliegenden Ausführungsform über verschiedene Zwischenstufen. Das CO₂ wird mittels Wasserstoff zu einem intermediären Kohlenstoffmonooxid reduziert und anschließend durch weitere Zugabe von Wasserstoff zu Methanol reduziert. Die Reduktion kann mittels Katalyse erfolgen. Die Reduktion kann z. B. übergangsmetallkatalysiert, oder direkt elektrochemisch mit geeigneten Elektrokatalysatoren erfolgen ohne ein intermediäres Kohlenstoffmonooxid auszubilden. Bei dem Katalysator kann es sich um einen Übergangsmetallkatalysator handeln. Der Katalysator kann dabei ausgewählt sein aus der Gruppe bestehend aus Platin-, Nickel-, Eisen-, Silber-, Kupfer-Katalysatoren und Kombinationen davon. Beispielsweise kann der Katalysator ausgewählt sein aus der Gruppe bestehend aus Platin-, Nickel-, Eisen-, Silber-, und Kupferbasierten Phosphorverbindungen.

Die Synthese des Methylmethacrylates erfolgt durch Umsetzen von Ethylen, Kohlenstoffmonooxid und Methanol. Hierbei geht das Verfahren von Ethylen als Rohstoff aus, das homogen katalysiert mit Kohlenmonoxid und Methanol in einem Schritt carboxymethyliert wird. Hierbei entsteht Propionsäuremethylester als Zwischenprodukt. Die Propionsäuremethylesterverbindung wird im nächsten Schritt in der Gasphase an einem speziellen Kontakt mit Formaldehyd aldolisiert und dehydratisiert, wobei direkt MMA entsteht.

Das Formaldehyd wird in der vorliegenden Ausführungsform durch ein mehrstufiges Verfahren hergestellt. Dabei wird CO₂ mit Wasserstoff zu Kohlenstoffmonooxid und Wasserstoff umgesetzt, aus dem Methanol entsteht. Das Methanol reagiert mit Sauerstoff unter Abspaltung von Wasser zu Formaldehyd.

Fig. 3b zeigt schematisch die Umsetzung von MMA zu PMMA. Dabei wird MMA in einer radikalischen Reaktion mit Dibenzoylperoxid (DBPO) oder Azoisobutyronitril (AiBN) zu PMMA umgesetzt.

### Ausführungsbeispiel 1

### 1.) Bildung von Ethylen aus CO₂

Elektrochemische Reduktion von CO₂ am Ni₂P/Fe₂P-Katalysator: Die elektrochemische Reduktion des CO₂ wurde durch die Anlagerung des Kohlenstoffdioxids über die Sauerstoffatome unter einer gleichzeitigen Hydridanbindung an die katalytisch aktive Ni₂P-Oberfläche initiiert. Durch eine Protonierung dieser Spezies wurde das Formaldehyd als Zwischenprodukt generiert. Das Formaldehyd reagierte im Anschluss mit zwei weiteren Formaldehydspezies über C-C-Kupplungsreaktionen an der katalytisch aktiven Elektrodenoberfläche über ein Glycoaldehyd zum Glycerinaldehyd (2,3-Dihydroxypropanal).

### 1.A. Herstellung der Elektrolysezelle:

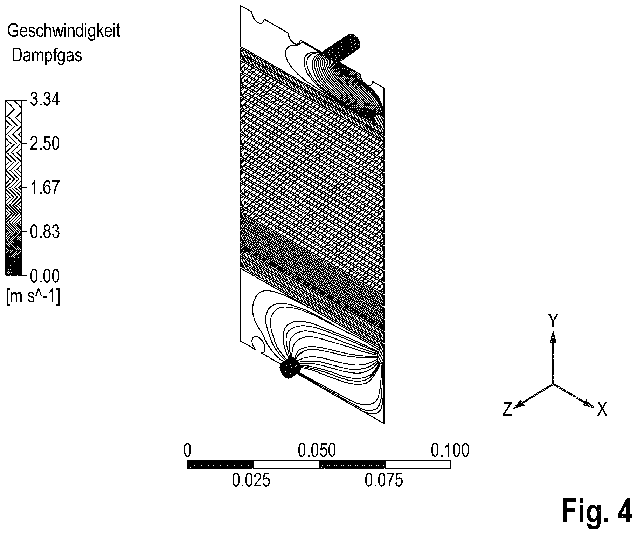

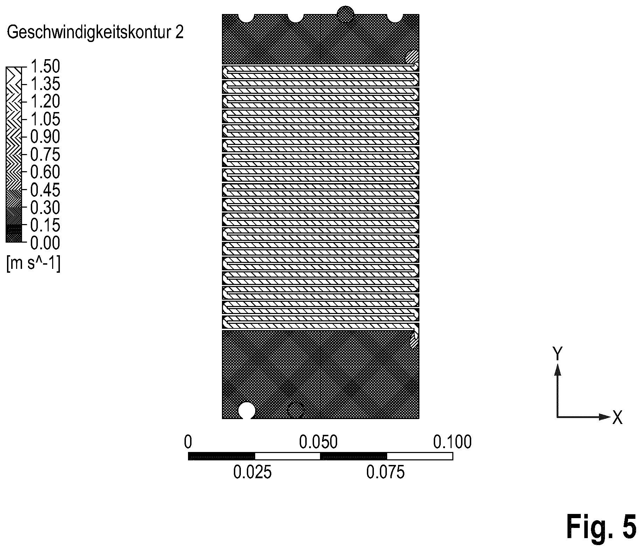

Bei der elektrochemischen Reduktion von CO₂ in Monoethylenglykol wurde aufgrund der vergleichsweise langsamen Diffusionsprozesse sowie der geringen Löslichkeit des CO₂ in Wasser eine Gasdiffusionselektrode präferiert. Das Grundgerüst kann wahlweise aus Titan oder Edelstahllegierungen mit einer typischen Dicke von 2 mm bestehen, in welchem strömungsgerechte Nuten mit einer Tiefe von 0,8 mm eingebracht werden. Ein exemplarisches strömungsgerechtes Profil ist in Fig. 4 und Fig. 5 dargestellt:

| | |
|---|---|
| Maximale Geschwindigkeit (m/s): | 3,717 |
| Durchschnittsgeschwindigkeit (m/s): | 1,592 |
| Strömungsgleichmäßigkeit (umax/uavg): | 2,3 |
| Verlust aktive Oberfläche: | 32 % |
| Nutenanzahl: | 35, l = 78 mm, b = 1 mm |

Die Reaktion verläuft vorzugsweise in einer Flusszelle, bei der als Anolyt 1 M KOH und als Katholyt 0,5 M KHCO₃ verwendet wird. Die Separatormembran kann beispielsweise aus Nafion^{™} bestehen.

### 1.B. Herstellung der Elektrokatalysatoren:

Die Synthese des Katalysatormaterials erfolgte im Muffelofen unter einem definierten Temperatur-Zeitprofil. Hierzu wurden die Elemente stöchiometrisch nach ihren jeweiligen Anteilen eingewogen und in, unter Argon stehenden, abgeschmolzenen Quarzglasampullen temperiert. Exemplarisch wurde zur Herstellung der Verbindung Ni₂P 3,956 g Ni sowie 1,060 g P eingewogen, im Mörser zerkleinert sowie vermischt und in die evakuierte sowie unter Schutzgas stehende Ampulle gefüllt. Diese wurde im Anschluss abgeschmolzen und im Muffelofen temperiert. Das Temperaturprogramm kann exemplarisch wie folgt aufgebaut sein: RT -> 350 °C -> 450 °C ->550 °C -> 700 °C
Heizrate: 0,5°C/min.

Jede Stufe wurde jeweils für 6 h gehalten und die Endtemperatur wurde für 24 h gehalten.

Im Anschluss wurde das überschüssige Ni durch Rühren in 10 % HCl unter N₂ entfernt und anschließend mit destilliertem Wasser gewaschen. Die Bestimmung der Phasenreinheit erfolgt durch PXRD (Pulverdiffraktometrie).

Die hergestellten Katalysatormaterialien wurden auf das Trägermaterial mithilfe eines geeigneten Bindermaterials aufgebracht.

### 1.C. Elektrochemische Synthese:

Die elektrochemische Synthese erfolgte unter einem initialen Screening des Katalysators mithilfe der Cyclovoltammetrie sowie elektrochemischer Impedanzspektroskopie. Im Anschluss wurde die detektierte benötigte Spannung potentiostatisch aufgebracht sowie gehalten und die elektrochemische Flusszelle in einem definierten Zeitfenster durch Pulsation mit erhöhter Spannung gereinigt. Das synthetisierte Produktgemisch besteht aus Monoethylenglykol (MEG), Wasser sowie anderen Verunreinigungen. Das MEG wurde im Anschluss über Elektrodeionisation sowie Destillation aufgereinigt und abgetrennt.

### 1.D. Corey-Winter-Eliminierung:

Die Corey-Winter-Eliminierung führte zur Ausbildung des Ethylens und wurde durch einen nucleophilen Angriff der Diolsauerstoffe am Thiocarbonylkohlenstoff initiiert. Unter Abspaltung von Chlorwasserstoff wurde das cyclische Thiocarbonat gebildet. Anschließend erfolgte ein nucleophiler Angriff des Corey-Hopkins-Reagenz an das Schwefelatom unter Bildung eines Carbanion. Der Thiophosphorsäureester wurde unter der Ausbildung eines cyclischen Carbens abgespalten, welches unter CO₂-Abspaltung das Ethylen als Reaktionsprodukt ausgebildet hatte.

Für die Corey-Winter-Eliminierung wurde 1,0 eq. vom MEG in Dichlormethan (DCM) sowie 2,4 eq. 4-Dimethylaminopyridin (4-DMAP) bei 0 °C gelöst. Der Lösung wurde im Anschluss 1,2 eq. Thiophosgen zugegeben und das Reaktionsgemisch für eine Stunde gerührt. Im Anschluss wurde das Reaktionsprodukt säulenchromatographisch über SiO₂ isoliert und das Lösungsmittel abdestilliert. Anschließend reagierte das Thiocarbonat mit Trimethylphosphid in Tetrahydrofuran (THF) bei 111 °C für 24 h. Das Reaktionsprodukt (Ethylen) wurde über eine Gaswaschflasche geleitet und aufgefangen.

### 2.) Bildung von CO aus CO₂

Das CO₂ kann mittels Wasserstoff zu einem intermediären Kohlenstoffmonooxid reduziert werden. Hierbei erfolgte die Umsetzung des Kohlenstoffdioxids zu Kohlenstoffmonooxid mithilfe übergangsmetallkatalysierter Reaktionen.

Die Reduktion kann mittels Katalyse erfolgen. Die Reduktion kann z. B. übergangsmetallkatalysiert oder direkt elektrochemisch mit geeigneten Elektrokatalysatoren erfolgen. Bei dem Katalysator kann es sich um einen Übergangsmetallkatalysator handeln. Der Katalysator kann dabei ausgewählt sein aus der Gruppe bestehend aus Nickel-, Eisen-, Silber- und Kupfer-Katalysatoren. Beispielsweise kann der Katalysator ausgewählt sein aus der Gruppe bestehend aus Nickel-, Eisen-, Silber-, und Kupfer-basierten Phosphorverbindungen. Die bevorzugte Variante besteht aus einer Gasdiffusionselektrode mit einem Elektrokatalysator, bestehend aus mindestens einem der folgenden Elemente: Zn, Co, Cr, Ni, Cu und Fe.

Hierzu wurden für die Herstellung der Arbeitselektrode Ag-Nanopartikel (20 nm) auf eine kohlenstoffbasierte Gasdiffusionsschicht aufgetragen. Die Katalysatortinte bestand aus Ag Nanopartikeln, einem lonomer und Isopropanol und wurde 15 min im Ultraschallbad homogenisiert. Diese wurde im Anschluss auf die Gasdiffusionsschicht aufgetragen und unter Stickstoff bei 70-90°C über 12-14 h getrocknet.

### 3.) Bildung von Methanol aus CO₂

Anschließend erfolgte durch weitere Zugabe von Wasserstoff die Bildung von Methanol. Die Reduktion kann mittels Katalyse erfolgen. Die Reduktion kann analog zur Bildung des Kohlenstoffmonooxids aus Schritt 2 übergangsmetallkatalysiert oder direkt elektrochemisch mit geeigneten Elektrokatalysatoren erfolgen. Der Katalysator kann dabei ausgewählt sein aus der Gruppe bestehend aus Platin-, Nickel-, Eisen-, Silber-, Kupfer-Katalysatoren und Kombinationen davon. Beispielsweise kann der Katalysator ausgewählt sein aus der Gruppe bestehend aus Platin-, Nickel-, Eisen-, Silber-, und Kupferbasierten Phosphorverbindungen. Dieses kann beispielsweise auf einem PtRu/C-Katalysator bei 1,25 V und 95°C erfolgen.

### 4.) Umsetzen von Ethylen, CO und Methanol

Die Synthese des Methylmethacrylates erfolgte durch Umsetzen von Ethylen, Kohlenstoffmonooxid und Methanol. Hierbei ging das Verfahren von Ethylen als Rohstoff aus, das homogen katalysiert mit Kohlenmonooxid und Methanol in einem Schritt carboxymethyliert wurde. Hierbei entstand Propionsäuremethylester als Zwischenprodukt. Die Propionsäuremethylesterverbindung wurde im nächsten Schritt in der Gasphase an einem speziellen Kontakt mit Formaldehyd aldolisiert und dehydratisiert, wobei direkt MMA entstanden ist.

### 5.) Polymerisation zum Polymethylmethacrylat (PMMA)

PMMA wird großtechnisch häufig radikalisch durch Substanz-, Emulsions-, oder Suspensionspolymerisation hergestellt. Auf solche Weise produziertes PMMA ist ataktisch und völlig amorph. Die Polymerisation wurde mit sogenannten Radikalbildnern wie beispielsweise Dibenzoylperoxid (DBPO) oder Azo-bis-(isobutylonitril) (AiBN) initiiert. Folgend verlief das Kettenwachstum radikalisch bis Rekombinationsreaktionen eingetreten sind. Eine anionische Polymerisation (einschließlich Methoden der lebenden Polymerisation) von PMMA ist ebenfalls möglich.

### Bezugszeichenliste

- 100: Verfahren zur Herstellung von Polymethylmethacrylat
- 101: Vorlegen von Ethylen
- 102: Vorlegen von Kohlenstoffmonooxid
- 103: Vorlegen von Methanol
- 104: Umsetzen von Ethylen, Kohlenstoffmonooxid und Methanol zu Methylmethacrylat
- 105: Polymerisieren von Methylmethacrylat zu Polymethylmethacrylat

## Patentansprüche

1. Verfahren zur Herstellung von Methylmethacrylat umfassend die Schritte:
- Vorlegen von Ethylen,
- Vorlegen von Kohlenstoffmonooxid,
- Vorlegen von Methanol, und
- Umsetzen des Ethylens, des Kohlenstoffmonooxids und des Methanols zu Methylmethacrylat,
wobei das Methylmethacrylat Kohlendioxid aus der Atmosphäre umfasst.

2. Verfahren zur Herstellung von Polymethylmethacrylat umfassend die Schritte:
- Herstellen von Methylmethacrylat nach Anspruch 1, und
- Polymerisieren des Methylmethacrylats zu Polymethylmethacrylat.

3. Verfahren zur Herstellung von Methylmethacrylat nach Anspruch 1 oder Verfahren zur Herstellung von Polymethylmethacrylat nach Anspruch 2, wobei das Methanol aus Kohlendioxid aus der Atmosphäre gebildet ist.

4. Verfahren nach einem der Ansprüche 1 bis 3, wobei das Vorlegen von Ethylen den Schritt umfasst: elektrochemisches Reduzieren von Kohlendioxid zu Ethylen.

5. Verfahren nach einem der Ansprüche 1 bis 4, wobei das Vorlegen von Kohlenstoffmonooxid den Schritt umfasst: elektrochemisches Reduzieren von Kohlendioxid zu Kohlenstoffmonooxid.

6. Verfahren nach einem der Ansprüche 1 bis 5, wobei das Vorlegen von Methanol den Schritt umfasst: katalytisches Reduzieren von Kohlendioxid zu Methanol.

7. Verfahren nach Anspruch 6, wobei der Katalysator ausgewählt ist aus der Gruppe bestehend aus Platin-, Nickel-, Eisen-, Silber-, Kupfer-Katalysatoren und Kombinationen davon.

8. Verfahren nach einem der Ansprüche 1 bis 7, wobei das Umsetzen des Ethylen, des Kohlenstoffmonooxids und des Methanols zu Methylmethacrylat unter heterogener Katalyse erfolgt.

9. Verfahren nach Anspruch 8, wobei das Umsetzen unter Ausbildung von Propionsäuremethylester als Zwischenprodukt erfolgt.

10. Verfahren nach Anspruch 9, wobei der Propionsäuremethylester mit Formaldehyd zu Methylmethacrylat umgesetzt wird.

11. Methylmethacrylat oder Polymethylmethacrylat hergestellt nach einem Verfahren der Ansprüche 1 bis 10.

12. Verwendung von Methylmethacrylat oder Polymethylmethacrylat nach Anspruch 11 in der Automobilindustrie.

13. Verwendung von Methylmethacrylat oder Polymethylmethacrylat nach Anspruch 11 in Blinker- und Rückleuchtengläser, Reflektoren, Lichtleiter und Tür-/Säulenverkleidungen im Exterieur- und Interieurbereich.

14. Verwendung von Methylmethacrylat oder Polymethylmethacrylat nach Anspruch 11 in Polymerbeton, Industriefußböden, Verglasungen, Detailabdichtungen im Flachdach, Industrietorverglasungen (Plustherm-Systemverglasung), Sanitär- und Einrichtungsbauteile, Türfüllungen, Lampenschirme, als Resist (Fotolack), als Bestandteil von Resist (Fotolack) in der Foto- und Elektronenstrahllithographie zur Herstellung von Schaltkreisen und Leiterplatten, Flutlicht-Schilder, Leuchtenabdeckungen, Leuchtwerbung, Schauglas, Linsen, Fresnel-Linsen, Lichtwellenleiter, Scheiben, Hauben, Scheinwerferabdeckungen, Methylmethacrylatklebstoff für Verbindungen von Metallen und Kunststoffen, Schlagzeuge und Tastenbeläge von Klavieren.
